# EUROPEAN PATENT APPLICATION

(11) **EP 3 795 676 A1**
(43) Date of publication of application: **24.03.2021**
(21) Application number: 18918503.6
(22) Date of filing: 01.06.2018
(51) Int. Cl.: C12N 1/20, A61P 35/00, C12R 1/01

(54) **APPLICATION OF B. FRAGILIS OR AKKERMANSIA MUCINIPHILA IN PREPARATION OF DRUG FOR PREVENTING OR TREATING TUMOR**

(30) Priority: 18.05.2018 CN 201810479187
(71) Applicant: Revaissant (Shenzhen) Biosciences Co., Ltd., Guangdong 518172 (CN)
(72) Inventor: ZENG, Gucheng, Guangzhou, Guangdong 510275 (CN)
(74) Representative: Patrade A/S
(86) International application number: PCT/CN2018/089561
(87) International publication number: WO 2019/218401

(57) **Abstract**

Provided is a use of *Bacteroides fragilis* or *Akkermansia muciniphila* in the preparation of a drug for preventing or treating tumors, wherein the drug promotes infiltration or accumulation of CD8 positive cytotoxic T lymphocyte in tumor microenvironment. Also provided is a use of a pharmaceutical composition, foodstuff, health product or food additive in preventing and/or treating tumors, wherein the pharmaceutical composition, foodstuff, health product or food additive comprises *Bacteroides fragilis* or *Akkermansia muciniphila,* and promotes infiltration or accumulation of CD8 positive cytotoxic T lymphocytes in tumor microenvironment.

## Description

### FIELD OF TECHNOLOGY

The present invention relates to the technical field of biomedicine, particularly, it relates to a use of *Bacteroides fragilis* or *Akkermansia muciniphila* in the preparation of a drug for preventing and/or treating tumors.

### BACKGROUND

Cancer has become the "first killer" of human beings. It is predicable by *The Global Cancer Report 2014,* published by the World Health Organization (WHO) that the global cancer cases will increase rapidly, and will increase year by year from 14 million people in 2012 to 19 million people in 2025, and to 24 million in 2035. Every year, there are about 7 million new cancer patients worldwide, and about 5 million tumor patients die, indicating that one person dies from tumor every 6 seconds. Chemotherapy is currently recognized as the main treatment method, and its main objective is to kill cancer cells in the body. However, chemotherapeutic drugs also damage normal human cells while killing cancer cells. Breast cancer is currently the most common type of malignant tumors, and it is also the most common disease that takes away the health and life quality of women. Methods of treating breast cancer are mainly surgical treatment and chemotherapy. With the development of personalized treatment through genetic diagnosis, breast cancer patients have good treatment results. However, due to various reasons, most patients have unsatisfactory curative effect. Therefore, the research of anti-breast cancer drugs is of great significance. In the *Annual Report of the ASCO Cancer Research Progress in 2016* published by the American Society of Clinical Oncology (ASCO) on February 4, 2016, immunotherapy was named as the biggest progress in cancer research in 2015. Just as Dr. Julie M. Vose, the chairman of ASCO, said, *"Immunotherapy is the most revolutionary breakthrough in the cancer field, and this new therapy not only can improve the lives of patients, but also give the direction for future research*". Current tumor immunotherapy will become the fourth major cancer treatment after surgery, radiotherapy and chemotherapy. Therefore, it has become a worldwide research hotspot to develop safe, low-priced, highly effective and low side-effect cancer immune drugs.

Currently, there are two tumor immunotherapeutic technologies showing good clinical effects. The first one is an adoptive cellular immunotherapy, which plays a role in anti-cancer treatment by obtaining the immune cells in body of the patients, then inducing to cells with killing effects, for example, chimeric antigen receptors T cell (CAR-T), etc., based upon the property of tumor target antigen, followed by transfusing the cells with killing effects back into the body. The second one is an antibody-targeted therapy, wherein targeted drugs inhibit cancer cells by interacting with a specific molecule target that is necessary during the growth or metastasis of tumors. Nowadays, there are antibody drugs for blocking T cell exhausted molecule such as CTLA4, PD-1, PD-L1, etc. Although these blocking antibody tumor treatments show clinical effects on some kinds of tumors for some patients, the blocking antibody drugs have low or no effects for a large proportion of patients, and a lack of the infiltration and accumulation of CD8 positive cytotoxic T lymphocytes (CD8⁺ T cells, for short) in tumor microenvironment is one of key reasons for the poor immunotherapeutic effect. How to promote the infiltration or accumulation of CD8⁺ T cells in tumor microenvironment becomes a key scientific and technical problem that needs to be solved urgently in tumor immunotherapy, considering that CD8⁺ T cells do not only have a key function of killing tumor cells directly but also improve efficacy of the immunotherapy by significantly enhancing the patient' responses to immunotherapy techniques, for example CAR-T and antibody drugs for blocking T cell exhausted molecule.

Uses of bacteria in cancer treatment can be traced back to the late nineteenth century, and there are even earlier reports on efficacy of bacteria in the treatment for cancer. Probiotics are active microorganisms that are beneficial to the host, and after probiotics are ingested into the human or animal body, they can settle on intestinal mucosa, establish intestinal microbiota and prevent harmful microorganism from adhering thereto. Probiotics also can keep people or animal healthy by maintaining natural intestinal microbiota and promoting the formation of healthy and viable microbial preparation for individual organisms. Currently, more and more researchers have focused on probiotics and gradually realized their powerful therapeutic effects. A lot of the latest works about bacteriotherapy for cancer focus on non-pathogenic strains. *Bifidobacterium* is a non-pathogenic and obligate anaerobic bacterium, and has been successfully used for targeting tumors and used as a therapeutic carrier, but without showing oncolysis. In recent years, some researches apply *Escherichia coli* and *pneumobacillus* for cancer/tumor of intestine and lung, respectively, as "site-specific immunomodulators" which play a more significant role in inhibiting tumor growth. However, uses of probiotics or intestinal bacteria for promoting the infiltration and accumulation of CD8⁺ T cell in tumor microenvironment haven't been reported.

*Bacteroides fragilis* is a Gram-negative, rod-shaped, non-motile, and non-spore-forming obligate anaerobic bacterium, having obtuse and hyperchromatic ends as well as a capsule. The Bacteroides fragilis can be classified into an enterotoxigenic type and a non-enterotoxigenic type. As a part of the normal intestinal flora of humans and animals, *Bacteroides fragilis* mainly exists in the colon, and besides, it can also colonize and grow in the respiratory tract, the gastrointestinal tract and the urogenital tract. Numerous researches have shown that *Bacteroides fragilis* has a good effect on the prevention and treatment of acute and chronic enteritis, dysbacteriosis, upper respiratory infection and neurosis, etc.

*Akkermansia muciniphila* (phylum *Verrucomicrobia*) is an anaerobic, atrichous, non-spore-forming, non-motile, Gram-negative, and oval-shaped gut bacterium, with a certain anaerobic ability. *Akkermansia muciniphila,* accounting for 1-3% of the total amount of gut microorganism, is one of the dominant intestinal flora of human, and the *Akkermansia muciniphila* colonized in mucous layer can specifically degrade mucoprotein. Current studies have found that *Akkermansia muciniphila* colonization abundance in humans is negatively correlated with obesity and type 2 diabetes, and *Akkermansia muciniphila* colonization is important for metabolisms in organisms.

However, uses of intestinal bacterium comprising *Bacteroides fragilis* and/or *Akkermansia muciniphila* for promoting the infiltration and/or accumulation of CD8⁺ T cells in tumor microenvironment to prevent and/or treat tumors haven't been reported.

### SUMMARY

In view of the difficulty of lacking the infiltration and/or accumulation of CD8 positive cytotoxic T lymphocytes (CD8⁺ T cells, for short) in tumor microenvironment for current tumor immunotherapy, it is to be provided a drug which can promote the infiltration or accumulation of CD8⁺ T cells in tumor microenvironment.

In order to achieve the above-mentioned objective, the present invention provides a use of *Bacteroides fragilis* (*B. fragilis*) or *Akkermansia muciniphila* in the preparation of a drug for preventing and /or treating tumors, wherein the drug promotes the infiltration or accumulation of CD8⁺ T cells in tumor microenvironment.

Preferably, the *Bacteroides fragilis* or *Akkermansia muciniphila* is any one selected from live bacterium of *Bacteroides fragilis* or *Akkermansia muciniphila*; genetically recombined, altered or modified, attenuated, chemically treated, physically treated, or inactivated *Bacteroides fragilis or Akkermansia muciniphila*; lysate of *Bacteroides fragilis* or *Akkermansia muciniphila*; and/or culture supernatant of *Bacteroides fragilis* or *Akkermansia muciniphila.*

Preferably, the tumors described in the present invention may be various solid tumors, for example, but are not limited to, breast cancer, or any one or more of liver, lung, skin, oral, esophagus, stomach, intestine, kidney, prostate, brain, nervous system, bladder, lymph, and pancreas tumors, such as solid tumors, for example lung cancer, melanoma tumor, liver cancer, prostate cancer, fibrosarcoma, bladder sarcoma, and glioma, etc.

The present invention also provides a method of promoting the infiltration and/or accumulation of CD8 positive cytotoxic T lymphocytes in tumor microenvironment to prevent and/or treat tumors.

Preferably, the *Bacteroides fragilis* or *Akkermansia muciniphila* is any one selected from live bacterium of *Bacteroides fragilis* or *Akkermansia muciniphila*; genetically recombined, altered or modified, attenuated, chemically treated, physically treated, or inactivated *Bacteroides fragilis* or *Akkermansia muciniphila*; lysate of *Bacteroides fragilis* or *Akkermansia muciniphila*; and/or culture supernatant of *Bacteroides fragilis* or *Akkermansia muciniphila.*

In some examples, the method of promoting the infiltration and/or accumulation of CD8⁺ T cells in tumor microenvironment to prevent and/or treat tumors is applied in combination with other methods of promoting the infiltration and/or accumulation of CD8⁺ T cells in tumor microenvironment. In some examples, the other methods of promoting the infiltration and/or accumulation of CD8⁺ T cells in tumor microenvironment to prevent and/or treat tumors include but are not limited to, chemotherapy, reflexotherapy, gene therapy, surgery, or a combination thereof.

The present invention further provides a therapeutic and prophylactic composition comprising *Bacteroides fragilis* or *Akkermansia muciniphila.* In some examples, the therapeutic and prophylactic composition includes *Bacteroides fragilis* or *Akkermansia muciniphila.* In some examples, the therapeutic and prophylactic composition does not include other microbial strains. In one aspect, the *Bacteroides fragilis* or *Akkermansia muciniphila* can inhibit tumor growth. In another aspect, the tumor is a solid tumor. In some examples, the tumor includes but is not limited to breast cancer.

According to one aspect of the present invention, the present invention further provides a pharmaceutical composition for preventing and/or treating tumors, wherein the pharmaceutical composition comprises a pharmaceutically effective amount of *Bacteroides fragilis* or *Akkermansia muciniphila* and a pharmaceutically acceptable carrier thereof, and can promote the infiltration and/or accumulation of CD8⁺ T cells in tumor microenvironment. The *Bacteroides fragilis* or *Akkermansia muciniphila* is an active ingredient.

Preferably, in above pharmaceutical composition, the *Bacteroides fragilis* or *Akkermansia muciniphila* is any one selected from live bacterium of *Bacteroides fragilis* or *Akkermansia muciniphila*; genetically recombined, altered or modified, attenuated, chemically treated, physically treated or inactivated *Bacteroides fragilis* or *Akkermansia muciniphila*; lysate of *Bacteroides fragilis* or *Akkermansia muciniphila*; and/or culture supernatant of *Bacteroides fragilis* or *Akkermansia muciniphila.*

Preferably, in the above pharmaceutical composition, the pharmaceutical composition can be made in any one or more of pharmaceutically acceptable dosage forms, including but are not limited to, tablet, capsule, oral liquid or freeze-dried powders.

Preferably, in the above pharmaceutical composition, the pharmaceutical acceptable carrier is one of skim milk, lactose, glucose, sucrose, sorbitol, mannose, trehalose, starch, arabic gum, calcium phosphate, alginate, gelatin, calcium silicate, fine crystalline cellulose, polyvinylpyrrolidone, cellulose, water, syrup, methyl cellulose, methyl hydroxybenzoate, propyl hydroxybenzoate, talc, magnesium stearate, or mineral oil, or a mixture thereof.

In order to better achieve the above-mentioned objective, the present invention further provides a foodstuff for treating and/or preventing tumors, wherein the foodstuff includes *Bacteroides fragilis* or *Akkermansia muciniphila,* and can promote the infiltration and/or accumulation of CD8 positive cytotoxic T lymphocytes in tumor microenvironment.

Preferably, in the above foodstuff, the *Bacteroides fragilis* or *Akkermansia muciniphila* is any one selected from live bacterium of *Bacteroides fragilis* or *Akkermansia muciniphila*; genetically recombined, altered or modified, attenuated, chemically treated, physically treated, or inactivated *Bacteroides fragilis* or *Akkermansia muciniphila*; lysate of *Bacteroides fragilis* or *Akkermansia muciniphila*; and/or culture supernatant of *Bacteroides fragilis* or *Akkermansia muciniphila.*

In order to better achieve the above-mentioned objective, present invention further provides a food additive for treating and/or preventing tumors, wherein the food additive includes *Bacteroides fragilis* or *Akkermansia muciniphila,* and can promote the infiltration and/or accumulation of CD8 positive cytotoxic T lymphocytes in tumor microenvironment.

Preferably, in the above food addictive, the *Bacteroides fragilis* or *Akkermansia muciniphila* is any one selected from live bacterium of *Bacteroides fragilis* or *Akkermansia muciniphila*; genetically recombined, altered or modified, attenuated, chemically treated, physically treated, or inactivated *Bacteroides fragilis* or *Akkermansia muciniphila*; lysate of *Bacteroides fragilis or Akkermansia muciniphila*; and/or culture supernatant of *Bacteroides fragilis* or *Akkermansia muciniphila.*

In order to better achieve the above-mentioned objective, present invention further provides a food additive for treating and/or preventing tumors, wherein the health product includes *Bacteroides fragilis* or *Akkermansia muciniphila,* and can promote the infiltration and/or accumulation of CD8 positive cytotoxic T lymphocytes in tumor microenvironment.

Preferably, in the above health product, the *Bacteroides fragilis* or *Akkermansia muciniphila* is any one selected from live bacterium of *Bacteroides fragilis* or *Akkermansia muciniphila*; genetically recombined, altered or modified, attenuated, chemically treated, physically treated, or inactivated *Bacteroides fragilis* or *Akkermansia muciniphila*; lysate of *Bacteroides fragilis or Akkermansia muciniphila*; and/or culture supernatant of *Bacteroides fragilis* or *Akkermansia muciniphila.*

In the invention, a transplanted tumor research method is applied to create a mouse breast cancer model, through which the effects of *Bacteroides fragilis* or *Akkermansia muciniphila* are detected and identified. Through experiments, the invention proves that the *Bacteroides fragilis* or *Akkermansia muciniphila* can significantly inhibit breast cancer from surviving in vitro and can effectively inhibit the growth of transplanted tumors in mice, indicating that it is important to develop and apply *Bacteroides fragilis* or *Akkermansia muciniphila* in clinical treatment of tumors.

### BRIEF DESCRIPTION

Figure 1 is a schematic flow diagram of an experiment of detecting the effect of *Bacteroides fragilis* and inactivated *Bacteroides fragilis* in promoting the accumulation of CD8⁺ T cells in tumor microenvironment and in treatment in a mouse breast cancer model.
Figure 2 is a typical analysis graph of flow cytometry of one mouse in each group after *Bacteroides fragilis* and inactivated *Bacteroides fragilis* are administrated to mice implanted with breast cancer cells, wherein right quadrant indicates CD8⁺ T cells, and figures of the right quadrant show the percentage of CD8⁺ T cells in total cells in tumor microenvironment.
Figure 3 is a statistical analysis graph of the percentage of CD8⁺ T cells in total cells in tumor microenvironment after *Bacteroides fragilis* and inactivated *Bacteroides fragilis* are administrated to mice implanted with breast cancer cells.
Figure 4 is a comparison diagram of tumor size of breast cancer of mice after being treated with *Bacteroides fragilis* and inactivated *Bacteroides fragilis.*
Figure 5 is a statistical analysis graph of the comparison diagram of tumor size of breast cancer in mice after being treated with *Bacteroides fragilis* and inactivated *Bacteroides fragilis*.
Figure 6 is a schematic flow diagram of an experiment of detecting the effect of *Akkermansia muciniphila* in promoting the accumulation of CD8⁺ T cells in tumor microenvironment and in treatment in a mice breast cancer model.
Figure 7 is a typical analysis graph of flow cytometry of one mouse in each group after *Akkermansia muciniphila* is administrated to mice implanted with breast cancer cells, wherein right quadrant indicates CD8⁺ T cells, and figures of the right quadrant show the percentage of CD8⁺ T cells in total cells in tumor microenvironment.
Figure 8 is a statistical analysis graph of the percentage of CD8⁺ T cells in total cells in tumor microenvironment after *Akkermansia muciniphila* is administrated to mice implanted with breast cancer cells.
Figure 9 is a comparison diagram of tumor size of breast cancer of mice after being treated with *Akkermansia muciniphila.*
Figure 10 is a statistical analysis graph of the comparison diagram of tumor size of breast cancer of mice after being treated with *Akkermansia muciniphila.*

### DETAILED DESCRIPTION

The present invention will be further described below with reference to the accompanied figures and examples. It should be pointed out that the *Bacteroides fragilis* or *Akkermansia muciniphila* for treating and/or preventing tumors in the present invention, or a pharmaceutical composition, foodstuff, health product or food additive containing the *Bacteroides fragilis* or *Akkermansia muciniphila* of the present invention is applied to the indications described above and exhibits the functions describe above, after being administrated to the subject. All dosage forms within the scope of the present invention have been tested, and their small parts are described hereinafter in the examples only for illustration, however, which should not be understood as a limitation of the present invention.

*Bacteroides fragilis* or *Akkermansia muciniphila* referred in the present invention includes but is not limited to any one selected from live bacterium of *Bacteroides fragilis* or *Akkermansia muciniphila*; genetically recombined, altered or modified, attenuated, chemically treated, physically treated, or inactivated *Bacteroides fragilis* or *Akkermansia muciniphila*; lysate of *Bacteroides fragilis* or *Akkermansia muciniphila*; and/or culture supernatant of *Bacteroides fragilis* or *Akkermansia muciniphila.*

The tumor is a solid tumor. In some examples, the tumor includes but is not limited to breast cancer.

The present invention further provides an anti-tumor pharmaceutical composition containing a pharmaceutically effective amount of *Bacteroides fragilis* or *Akkermansia muciniphila,* wherein the so-called "pharmaceutically effective amount" is 10⁶-10¹⁰ CFU, preferably 10⁹ CFU. The *Bacteroides fragilis* or *Akkermansia muciniphila* is any one selected from live bacterium of *Bacteroides fragilis* or *Akkermansia muciniphila*; genetically recombined, altered or modified, attenuated, chemically treated, physically treated, or inactivated *Bacteroides fragilis* or *Akkermansia muciniphila*; lysate of *Bacteroides fragilis* or *Akkermansia muciniphila*; and/or culture supernatant of *Bacteroides fragilis* or *Akkermansia muciniphila.* The pharmaceutical composition includes but is not limited to tablet, capsule, oral liquid, or frizzed-dried powders. The pharmaceutically acceptable carrier includes but is not limited to one or more of skim milk, lactose, glucose, sucrose, sorbitol, mannose, trehalose, starch, arabic gum, calcium phosphate, alginate, gelatin, calcium silicate, fine crystalline cellulose, polyvinylpyrrolidone, cellulose, water, syrup, methyl cellulose, methyl hydroxybenzoate, propyl hydroxybenzoate, talc, magnesium stearate, or mineral oil.

The *Bacteroides fragilis* or *Akkermansia muciniphila* of the present invention can be made into foodstuffs, health products, or food additives, etc. The foodstuffs, health products or food additives contain any one selected from live bacterium of *Bacteroides fragilis* or *Akkermansia muciniphila*; genetically recombined, altered or modified, attenuated, chemically treated, physically treated, or inactivated *Bacteroides fragilis* or *Akkermansia muciniphila*; lysate of *Bacteroides fragilis* or *Akkermansia muciniphila*; and/or culture supernatant of *Bacteroides fragilis* or *Akkermansia muciniphila.* The foodstuffs, health products or food additives are used for treating and/or preventing tumors.

### Example 1 Culture of Bacteroides fragilis

### Culture Method

Step 1: A cryopreserved *Bacteroides fragilis* strain (purchased from ATCC official website) was taken and then 200 uL of Tryptone Soya Broth (TSB) culture medium was added to redissolve it to obtain a bacterial solution. Subsequently, 20 uL of the bacterial solution was pipetted and streaked on a blood agar plate. After an air exhaustion by an anaerobic jar gassing system, the agar plate was placed in an incubator and incubated anaerobically at 37° C for 48 h;
Step 2: A monoclonal colony was selected to inoculate in 10 mL of TSB culture medium, and incubated anaerobically at 37° C for 12 h;
Step 3: 1% (v/v) of strain was inoculated in 500 ml of TSB culture medium in a flask and incubated anaerobically at 37° C for 48 h;
Step 4: After the bacterial solution was collected, it was centrifuged at 6000 rpm for 10 min, washed twice with saline. Finally, the bacterial sludge was redissolved with saline for later use and viable bacterium were counted.

### Example 2 Culture of Akkermansia muciniphila

### Culture Method

Step 1: A cryopreserved *Akkermansia muciniphila* strain (purchased from official website of ATCC) was taken and 200 uL of Tryptone Soya Broth (TSB) culture medium was added to redissolve it to obtain a bacterial solution. Subsequently, 20 uL of the bacterial solution was pipetted and streaked on a blood agar plate. After an air exhaustion by an anaerobic jar gassing system, the agar plate was placed in an incubator and incubated anaerobically at 37° C for 48 h;
Step 2: A monoclonal colony was selected to inoculate in 10 mL of TSB culture medium, and incubated anaerobically at 37° C for 12 h;
Step 3: 1% (v/v) of strain was inoculated in 500 ml of TSB culture medium in a flask and incubated anaerobically at 37° C for 48 h;
Step 4: After the bacterial solution was collected, it was centrifuged at 6000 rpm for 10 min, washed twice with saline. Finally, the bacterial sludge was redissolved with saline for later use and viable bacterium were counted.

### Example 3 An experiment of effect of Bacteroides fragilis in promoting the infiltration and/or accumulation of CD8⁺ T cells in tumor microenvironment and in treating.

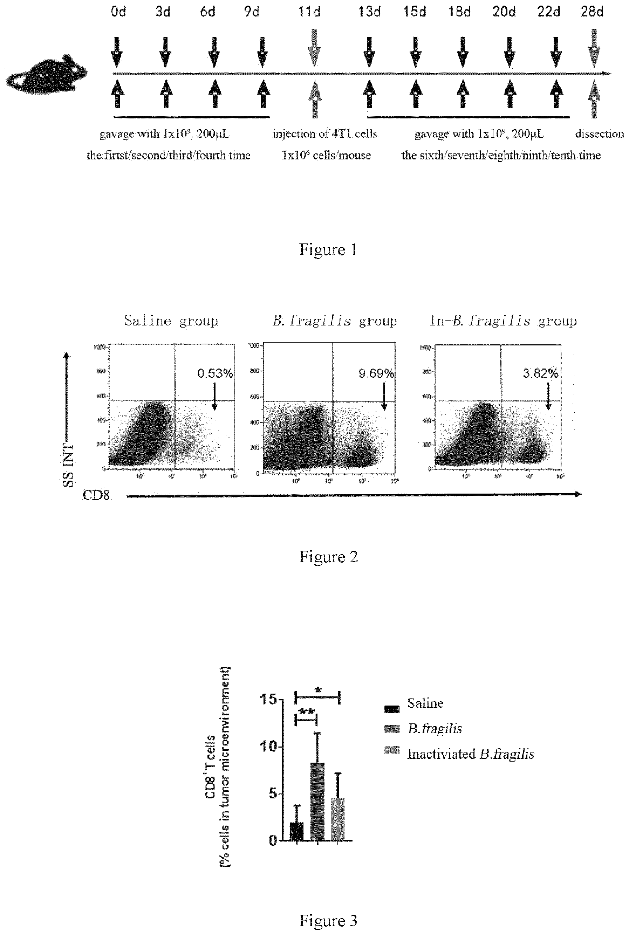

Figure 1 is a schematic flow diagram of an experiment for detecting the effect of *Bacteroides fragilis* and inactivated *Bacteroides fragilis* in promoting the accumulation of CD8⁺T cells in tumor microenvironment and in treatment.

### 1. Culture Method

A culture method of *Bacteroides fragilis* is the same as that in Example 1.

### 2. Sample Preparation

### 1) Preparation of a live strain of Bacteroides fragilis ZY-312

Step 1: A cryopreserved *Bacteroides fragilis* strain (purchased commercially) was taken and 200 uL of culture medium for cryopreserved strain was added to redissolve it to obtain a bacterial solution. Subsequently, 20 uL of the bacterial solution was pipetted and streaked on a blood agar plate. After an air exhaustion by an anaerobic jar gassing system, the agar plate was placed in an incubator and incubated anaerobically at 37° C for 48 h;
Step 2: A monoclonal colony was selected to inoculate in 10 mL of TSB culture medium, and incubated anaerobically at 37° C for 12 h;
Step 3: 1% (v/v) of strain was inoculated in 500 ml of TSB culture medium in a flask and incubated anaerobically at 37°C for 48 h;
Step 4: After the bacterial solution was collected, it was centrifuged at 6000 rpm for 10 min, washed twice with saline. Finally, the bacterial sludge was redissolved with saline for later use and viable bacterium were counted.

### 2) Inactivated Bacteroides fragilis (In-B. fragilis)

The *Bacteroides fragilis* was heated in a water bath at 70° C for 30 min to obtain inactivated bacteria solution of *Bacteroides fragilis*.

### 3) Lysate of Bacteroides fragilis

The bacterial solution of *Bacteroides fragilis* was cultured, and an ultrasonication was performed by an ultrasonic machine for 2 seconds every 5 seconds, the total ultrasonication lasts for 20 minutes, a lysate of *Bacteroides fragilis* or *Akkermansia muciniphila* was obtained.

### 4) Supernate of Bacteroides fragilis

The bacterial solution of *Bacteroides fragilis* was cultured and centrifuged by a centrifuge at 6000 rpm for 10 min, to obtain supernate of *Bacteroides fragilis*.

### 3. An experiment of prevention and treatment effect of Bacteroides fragilis on tumor in mice

Experimental animal: Thirty-six BALB/c mice aged 3-6 weeks in a good mental state were purchased from the Experimental Animal Center of Sun Yat-sen University. The mice were randomly separated into three groups, 12 mice in each group, the three groups were the control group (saline), the live bacteria gavage group (*Bacteroides fragilis*), and the inactivated bacteria gavage group (inactivated *Bacteroides fragilis*) respectively. The three groups of mice were administered with *Bacteroides fragilis* and the control solution by gavage with the density of 10⁹ CFU, respectively, and their body weight were measured daily. After 4T1 mouse tumor (breast cancer) cells grew at logarithmic phase, the cells were digested with TE, and culture medium was neutralized. The cells were collected through centrifugation, and washed twice with DPBS to remove residual serum. After that, the cells were resuspended with DPBS. When the cell counting was completed, each mouse was subcutaneously inoculated with 10⁶ cells into right axilla and continually treated by gavage. Subsequently, tumor-bearing mice were killed. The tumor cells in situ were collected, and were detected by using flow cytometry to analyze the percentage of CD8+ T cells.

### Example 4 An experiment of effect of Akkermansia muciniphila in promoting the infiltration and/or accumulation of CD8⁺T cells in tumor microenvironment and treating tumors

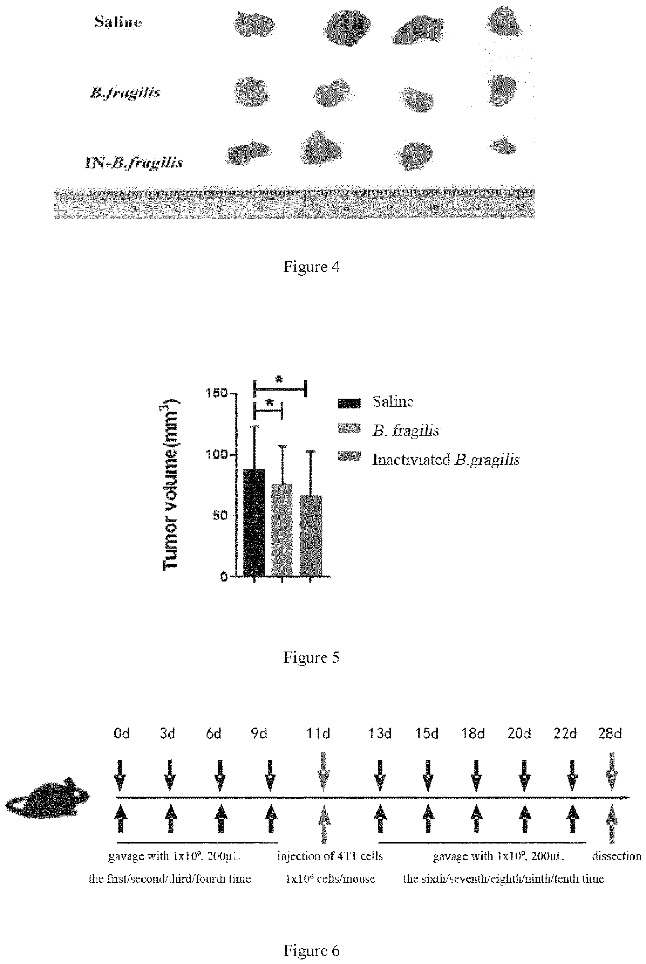

Figure 6 is a schematic flow diagram of an experiment of detecting the effect of *Akkermansia muciniphila* and inactivated *Akkermansia muciniphila* in promoting the accumulation of CD8⁺ T cells in tumor microenvironment and in treatment.

### 1. Culture Method

A culture method of *Akkermansia muciniphila* is the same as that in Example 2.

### 2. Sample Preparation

### 1)Preparation of a live strain of Akkermansia muciniphila

Step 1: A cryopreserved *Akkermansia muciniphila* strain (purchased commercially) was taken and 200 uL of culture medium for the cryopreserved strain was added to redissolve it to obtain a bacterial solution. Subsequently, 20 uL of the bacterial solution was pipetted and streaked on a blood agar plate. After an air exhaustion by an anaerobic jar gassing system, the agar plate was placed in an incubator and incubated anaerobically at 37° C for 48 h;
Step 2: A monoclonal colony was selected to inoculate in 10 mL of TSB culture medium, and incubated anaerobically at 37° C for 12 h;
Step 3: 1% (v/v) of strain was inoculated in 500 ml of TSB culture medium in a flask and incubated anaerobically at 37° C for 48 h;
Step 4: After the bacterial solution was collected, it was centrifuged at 6000 rpm for 10 min, washed twice with saline. Finally, the bacterial sludge was redissolved with saline for later use and viable bacterium were counted.

### 2) Inactivated Akkermansia muciniphila

The *Akkermansia muciniphila* was heated in a water bath at 70 ° C for 30 min to obtain inactivated *Akkermansia muciniphila.*

### 3) Lysate of Akkermansia muciniphila

The bacterial solution of *Akkermansia muciniphila* was cultured, and an ultrasonication was performed by an ultrasonic machine for 2 seconds every 5 seconds, the total ultrasonication lasts for 20 minutes, a lysate of *Akkermansia muciniphila* was obtained.

### 4) Supernate of Akkermansia muciniphila

The bacterial solution of *Akkermansia muciniphila* was cultured and centrifuged by a centrifuge at 6000 rpm for 10 min, to obtain a supernate of *Akkermansia muciniphila.*

### 3. An experiment of prevention and treatment effect of Akkermansia muciniphila on tumor in mice

Experimental animal: Twenty-four BALB/c mice aged 3-4 weeks in a good mental state were purchased from the Experimental Animal Center of Sun Yat-sen University. The mice were randomly separated into two groups, 12 mice in each group, the two groups were the control group (saline) and the live bacteria gavage group (*Akkermansia muciniphila*), respectively. The two groups of mice were administered with *Akkermansia muciniphila* and the control solution by gavage with the density of 10⁹ CFU, respectively, and their body weight were measured daily. After 4T1mouse tumor (breast cancer) cells grew at logarithmic phase, the cells were digested with TE, and culture medium was neutralized. The cells were collected through centrifugation, and washed twice with DPBS to remove residual serum. After that, the cells were resuspended with DPBS. When the cell counting was completed, each mouse was subcutaneously inoculated with 10⁶ cells into right axilla and continually treated by gavage. Subsequently, tumor-bearing mice were killed. The tumor cells in situ were collected, and were detected by using flow cytometry to analyze the percentage of CD8⁺ T cells.

### Analysis of test results

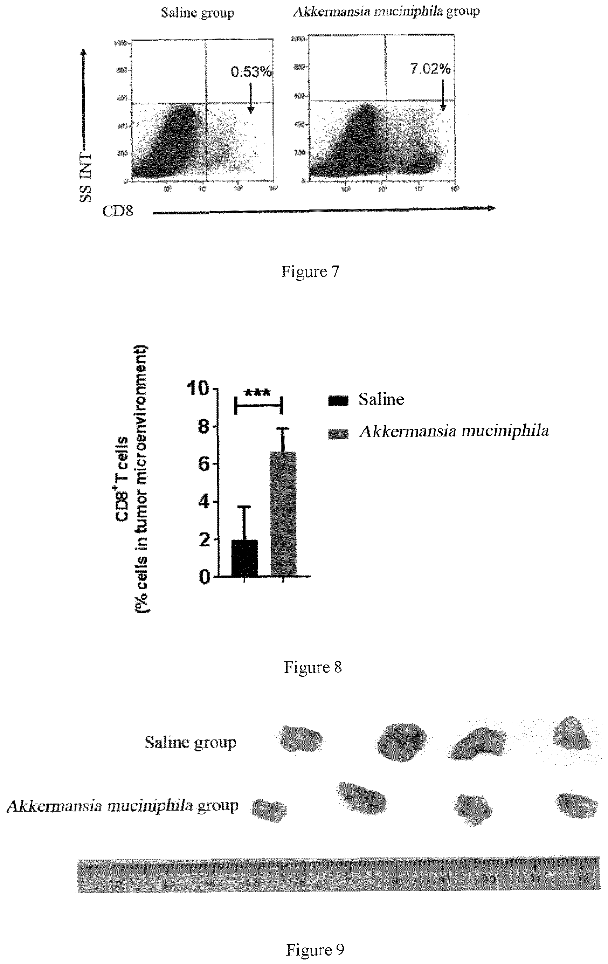

Figure 2 and Figure 7 show typical flow cytometry test results of each mouse and Figure 3 and Figure 8 show statistical results of multiple mice in each group.

Figure 2 is a typical analysis graph of flow cytometry of one mouse in each group after *Bacteroides fragilis* are administrated to mice implanted with breast cancer cells, wherein figures of the right quadrant show the percentages of CD8⁺ T cells in cells in tumor microenvironment. As shown from the quadrant graph in analysis graph of the flow cytometry, *Bacteroides fragilis* and inactivated *Bacteroides fragilis* increase the relative amount of the CD8⁺ T cells by about 20 times compared with the saline control group. Figure 3 is a statistical analysis graph of the percentage of CD8⁺ T cells in cells in tumor microenvironment after *Bacteroides fragilis* are administrated to mice implanted with breast cancer cells. As shown from the statistical graph, *Bacteroides fragilis* and inactivated *Bacteroides fragilis* significantly increase the amount of the CD8⁺ T cells in tumor microenvironment. In the statistical analysis graph, * represents student *t*-test p<0.05, ** represents student t-test p<0.01. p<0.05 represents statistically significant difference. There are 12 mice in each treated group.

Figure 7 is a typical analysis graph of flow cytometry of one mouse in each group after *Akkermansia muciniphila* is administrated to mice implanted with breast cancer cells, wherein figures of the right quadrant show the percentages of CD8⁺ T cells in cells in tumor microenvironment. As shown from the quadrant graph in analysis graph of the flow cytometry, *Akkermansia muciniphila* increases the relative amount of the CD8⁺ T cells by more than 13 times compared with the saline control group. Figure 8 is a statistical analysis graph of the percentage of CD8⁺ T cells in cells in tumor microenvironment after *Akkermansia muciniphila* is administrated to mice implanted with breast cancer cells. As shown from the statistical graph, *Akkermansia muciniphila* significantly increase the amount of the CD8⁺ T cells in tumor microenvironment. In the statistical analysis graph, *** represents student *t*-test p<0.001. p<0.001 represents statistically significant difference. There are 12 mice in each treated group.

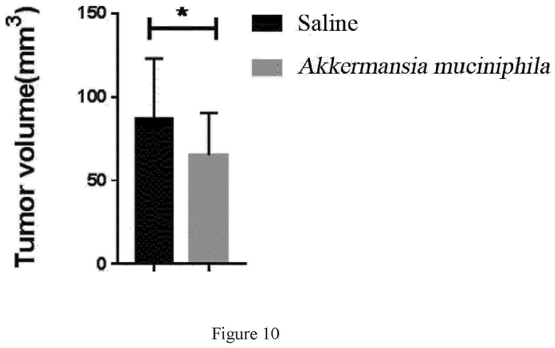

The results show that either *Bacteroides fragilis* and inactivated *Bacteroides fragilis* or *Akkermansia muciniphila* has a significant role in inhibiting the formation and growth of tumor in mice (Figures 4, 5, 9 and 10). In addition, the results of Figures 4, 5, 9 and 10 show the tumor size in mice treated with *Bacteroides fragilis* or *Akkermansia muciniphila* by gavage is significantly smaller than that in saline control group, indicating that *Bacteroides fragilis* and inactivated *Bacteroides fragilis* or *Akkermansia muciniphila* promotes the infiltration and/or accumulation of CD8⁺ T cell in tumor microenvironment to enhance anti-tumor effect in the body, and inhibits the tumor growth, having a good effect on the prevention and treatment of tumors, such as breast cancer.

The above content is a further detailed description of the technical solution in combination with the preferred embodiments of the present invention. It cannot be considered that the specific implementation of the present invention is limited to the above descriptions. For those skilled in the art, any simple deductions or replacements without departing from the inventive concept of the invention should all be regarded as belonging to the protection scope of the invention.

## Claims

1. Use of *Bacteroides fragilis* or *Akkermansia muciniphila* in the preparation of a drug for preventing or treating tumors, wherein the drug promotes infiltration and/or accumulation of CD8 positive cytotoxic T lymphocytes in tumor microenvironment.

2. The use according to claim 1, wherein the *Bacteroides fragilis* or *Akkermansia muciniphila* is any one selected from live bacterium of *Bacteroides fragilis* or *Akkermansia muciniphila*; genetically recombined, altered or modified, attenuated, chemically treated, physically treated, or inactivated *Bacteroides fragilis* or *Akkermansia muciniphila*; lysate of *Bacteroides fragilis or Akkermansia muciniphila*; and/or culture supernatant of *Bacteroides fragilis* or *Akkermansia muciniphila.*

3. The use according to claim 1 or 2, wherein the tumor is a solid tumor.

4. The use according to claim 1 or 2, wherein the tumor is breast cancer, or any one or more of liver, lung, skin, oral, esophagus, stomach, intestine, kidney, prostate, brain, nervous system, bladder, lymph, and pancreas tumors.

5. A pharmaceutical composition for preventing and/or treating tumors, comprising a pharmaceutically effective amount of *Bacteroides fragilis* or *Akkermansia muciniphila* and a pharmaceutically acceptable carrier thereof, and promoting infiltration and/or accumulation of CD8 positive cytotoxic T lymphocytes in tumor microenvironment.

6. The pharmaceutical composition according to claim 5, wherein the *Bacteroides fragilis or Akkermansia muciniphila* is any one selected from live bacterium of *Bacteroides fragilis* or *Akkermansia muciniphila*; genetically recombined, altered or modified, attenuated, chemically treated, physically treated, or inactivated *Bacteroides fragilis* or *Akkermansia muciniphila*; lysate of *Bacteroides fragilis* or *Akkermansia muciniphila*; and/or culture supernatant of *Bacteroides fragilis* or *Akkermansia muciniphila.*

7. A foodstuff for preventing and/or treating tumors, including *Bacteroides fragilis* or *Akkermansia muciniphila,* and promoting infiltration and/or accumulation of CD8 positive cytotoxic T lymphocytes in tumor microenvironment.

8. The foodstuff according to claim 7, wherein the *Bacteroides fragilis* or *Akkermansia muciniphila* is any one selected from live bacterium of *Bacteroides fragilis* or *Akkermansia muciniphila*; genetically recombined, altered or modified, attenuated, chemically treated, physically treated, or inactivated *Bacteroides fragilis* or *Akkermansia muciniphila*; lysate of *Bacteroides fragilis or Akkermansia muciniphila*; and/or culture supernatant of *Bacteroides fragilis* or *Akkermansia muciniphila.*

9. A food additive for preventing and/or treating tumors, including *Bacteroides fragilis* or *Akkermansia muciniphila,* and promoting infiltration and/or accumulation of CD8 positive cytotoxic T lymphocytes in tumor microenvironment.

10. The food additive according to claim 9, the *Bacteroides fragilis* or *Akkermansia muciniphila* is any one selected from live bacterium of *Bacteroides fragilis* or *Akkermansia muciniphila*; genetically recombined, altered or modified, attenuated, chemically treated, physically treated, or inactivated *Bacteroides fragilis* or *Akkermansia muciniphila*; lysate of *Bacteroides fragilis or Akkermansia muciniphila*; and/or culture supernatant of *Bacteroides fragilis* or *Akkermansia muciniphila.*
